Europäisches Patentamt

European Patent Office

Office européen des brevets

(11) Publication number: **0 325 763 B1**

# EUROPEAN PATENT SPECIFICATION

(45) Date of publication of patent specification: **01.07.92**

(51) Int. Cl.⁵: **C12Q 1/68**, G05D 23/19, C07H 21/00, B01J 19/00

(21) Application number: **88121108.0**

(22) Date of filing: **16.12.88**

(54) **Apparatus and method for amplification of nucleic acids.**

(30) Priority: **23.12.87 FI 875696**

(43) Date of publication of application:
**02.08.89 Bulletin 89/31**

(45) Publication of the grant of the patent:
**01.07.92 Bulletin 92/27**

(84) Designated Contracting States:
**AT BE CH DE ES FR GB GR IT LI LU NL SE**

(56) References cited:
EP-A- 0 134 622
EP-A- 0 223 618
EP-A- 0 236 069

NUCLEIC ACID RESEARCH, vol.16, no.12, 24 June 1988, IRL Press Ltd., Oxford (GB); N.S.FOULKES et al., pp.5687-5688

NUCLEIC ACID RESEARCH, vol.16, no.7, 11 April 1988, IRL Press Ltd, London (GB); F.ROLLO et al., pp.3105-3106

DNA, vol.7, 06 November 1988, Mary Ann Liebert, Inc., Publishers, New York, NY (US); H.U.WEIER et al., pp.441-447

(73) Proprietor: **ORION CORPORATION LIMITED**
**PL 65**
**SF-02101 Espoo(FI)**

(72) Inventor: **Kalkkinen, Nisse Erkki Juhani**
**Kaskimäki 20**
**SF-02780 Espoo(FI)**
Inventor: **Söderlund, Hans Erik**
**Salonkitie 19**
**SF-02940 Espoo(FI)**

(74) Representative: **Vossius & Partner**
**Siebertstrasse 4 P.O. Box 86 07 67**
**W-8000 München 86(DE)**

# Description

The present invention relates to an apparatus and a method for performing automated amplification of nucleic acids under standardized conditions. The invention also relates to a disposable part for the apparatus used in the method.

The amplification of nucleic acids is described in US -A- 4,683,194, 4,683,195 and 4,683,202, and in EP -A- 200 362, 229 701 and 237 362. In amplification, a reaction mixture which contains the target nucleic acid, at least two suitable primers, four different deoxynucleoside triphosphates, and DNA polymerase in a suitable buffer solution is first incubated at a suitable temperature in order to polymerize the DNA. Thereafter the double-stranded DNA formed in the polymerization is denatured by heating and the reaction mixture is cooled to a temperature at which the primers are capable of hybridizing with the target DNA. When necessary, the temperature of the reaction mixture is adjusted to a temperature optimal for the action of DNA polymerase, and DNA polymerase is added. The steps described above are repeated as many times as is necessary for producing the desired result.

Amplification has in general been performed manually by transferring the test tubes from one place to another dozens of times. The method is slow and cumbersome to perform. When thermolabile polymerase has been used, it has been necessary to open and close the tubes at intervals. Furthermore, in order to produce a homogenous reaction fluid, it has been necessary to centrifuge from the walls of the tubes the reaction fluid condensed during the cooling step. Thus it has not been possible to automate the process. It has been a further disadvantage that the reaction conditions have not necessarily remained quite the same during repeats. This has constituted a problem, especially when a thermostable enzyme has been used, since in spite its thermostability polymerase is readily destroyed if it is kept at a denaturation temperature for too long a time.

EP -A- 236 069 describes an apparatus in which the amplification of nucleic acids is performed under computer control by heating and cooling the reaction mixture in the same vessel. In this apparatus, only a thermostable polymerase can be used. In practice, a thermostable polymerase is active for the duration of only a few reaction cycles. It is difficult to heat and cool a reaction mixture with precision and with sufficient speed, since in addition to the temperature of the reactor and the reaction mixture in it, the temperature of the surrounding apparatus also has to be adjusted. The heat capacity of the surrounding apparatus for its part is inevitably considerable compared with the heat capacity of the small-volume (approximately 100 μl) reaction mixture. For this reason it easily occurs that the reaction mixture will be for too long a time under conditions disadvantageous for amplification. Problems arise in particular in the controlling of the denaturation temperature and time. If the temperature is too low, denaturation will not proceed in the manner desirable with respect to amplification. On the other hand, if the polymerase has to be at the denaturation temperature for too long a time, it will be destroyed.

The object of the present invention is to provide an apparatus and method eliminating the above-mentioned disadvantages. By using the apparatus of the present invention it is possible fully automatically, without separate steps requiring an operator, to perform the amplification of nucleic acids on one or several samples simultaneously, under standardized reaction conditions. In the apparatus according to the invention, the correct denaturation temperature and time can be adjusted with sufficient precision and speed. The method and apparatus according to the invention can be used for the amplification of nucleic acids regardless of whether the polymerase used is thermostable or not.

The invention relates to a method for performing the amplification of nucleic acids in an apparatus as defiend in the claims by incubating in a vessel, in order to perform polymerization, a reaction mixture which contains in a suitable buffer solution one or several single-stranded target nucleic acids, suitable primers, deoxynucleoside triphosphates and a polymerase, whereafter the reaction mixture is transferred, by a procedure suitable in the given case, into another vessel for the denaturation of the nucleic acids, from which vessel, after denaturation, the reaction mixture is transferred back into the original vessel, by using a suitable procedure. The amplification apparatus is regulated by maintaining at a temperature advantageous for the action of the polymerization enzyme the vessel in which the polymerization of the nucleic acid occurs, and by maintaining at a temperature advantageous for denaturation the vessel in which the denaturation is performed. The transferring of the reaction mixture from the denaturation vessel into the polymerization vessel is via a heat exchanger. In the heat exchanger the denatured reaction mixture is preferably cooled to a temperature at which the primers are capable of hybridizing with the target DNA.

The invention also relates to an apparatus for use in the method. The apparatus includes one or several, preferably parallel, pairs of vessels. A vessel pair is made up of two vessels which are interconnected or capable of being interconnected by means of one or several tubes. The apparatus may be provided with a separate heat exchanger via which at least one of the tubes passes, or a tube may as such serve as a heat exchanger. Each of the vessels is provided with a thermoregulator, for example a heat block provided with a thermostat. Furthermore, the apparatus includes a fluid-transfer system for transferring the reaction fluid from one vessel to another. The vessel in which the polymerization occurs may be equipped with a suitable dosing device for the adding of the polymerase and/or reagents.

The invention further relates to a disposable apparatus part which includes, in addition to the vessel pair and the tube connecting them, gas tubes leading to the vessels, which tubes can be used in the preferred embodiment of the apparatus according to the invention, described in greater detail below.

The method and apparatus according to the invention are illustrated in the following figures, in which

Figure 1 depicts the polymerization step and the general principle of the apparatus according to the invention,

Figure 2 depicts the step of transferring the fluid into the vessel in which the denaturation of DNA occurs,

Figure 3 depicts the step of denaturation of DNA,

Figure 4 depicts the transferring of the fluid back into the vessel in which the polymerization reaction occurs.

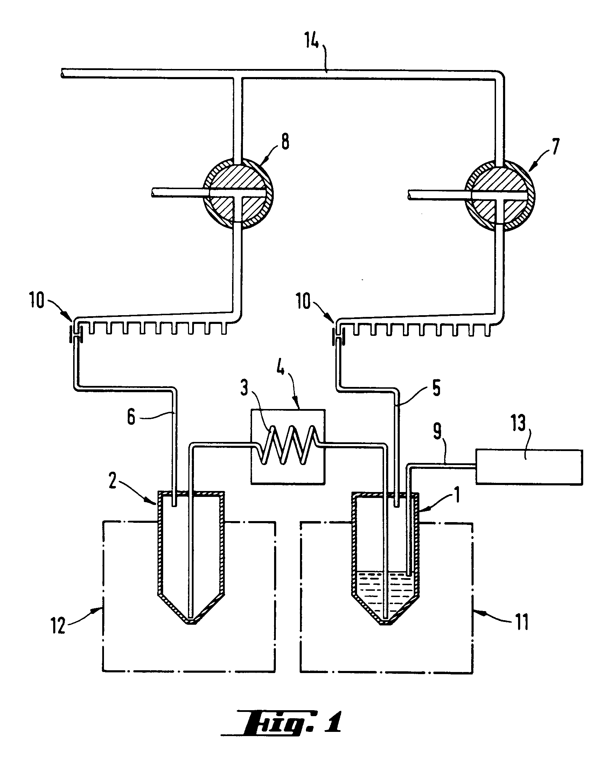

Figure 1 depicts one preferred embodiment, in which the reaction mixture is in the vessel 1 in which the polymerization reaction occurs. A tube 3 leads from vessel 1 to vessel 2. The tube 3 is advantageously a teflon tube having an inner diameter of 0.3-0.5 mm. In conjunction with the tube 3 there may be a separate heat exchanger 4. The heat exchanger 4 can also be substituted for simply by increasing the length of the tube. The vessels are completely airtight. The end of the tube 3 should in each vessel reach a point close to the vessel bottom in order that substantially the entire fluid volume could be transferred through the tube into the other vessel. For this reason it is advantageous to give the vessel bottom a downwards converging shape so that the fluids flow towards the converging depression. It is, of course, advantageous to place the end of the tube 3 in this depression. There are also gas tubes 5, 6 leading to each vessel 1, 2 respectively. These gas tubes are connected to a valve system made up of microprocessor-controlled valves, for example magnetic three-way valves 7 and 8. It is possible, when so desired, to connect via a valve the flow of gas to one of the vessels, at which time the valve of the gas tube leading to the other vessel must be deactivated so that excess gas can flow out into the surrounding air and the flow of fluids from one vessel to the other becomes possible. Examples of suitable gases are inert gases, for example, nitrogen and argon. Through a dosing device and a tube 9 belonging to the dosing device 13 it is possible, when necessary, to add polymerase or other reagents into the vessel 1. When several parallel samples are being amplified, similar vessel pairs made up vessels 1 and 2 can be connected via gas tubes 5 and 6 to gas distributors 10, which are controlled by valves 7 and 8. In Figure 1, the polymerization reaction is in progress, the magnetic valves 7 and 8 being deactivated, i.e. open to the outer air. By means of a thermoregulator (11) the vessel 1 is being kept at a temperature optimal for polymerization.

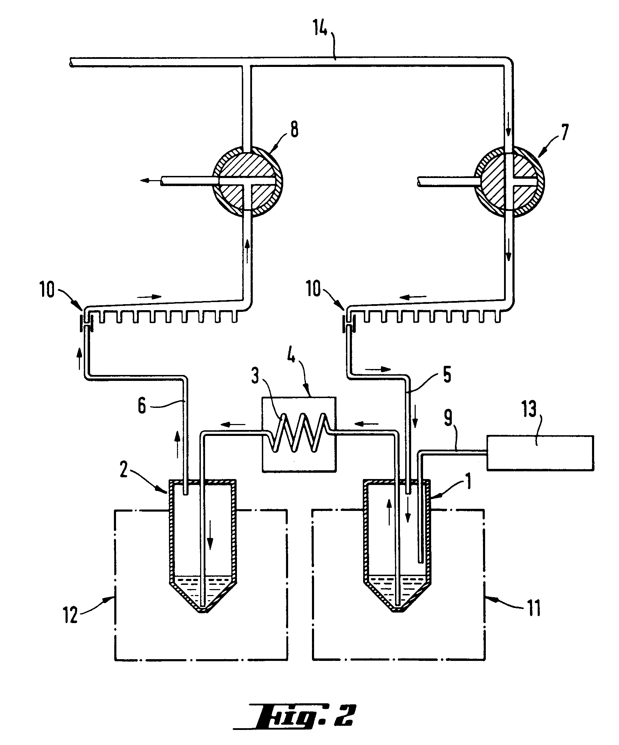

In Figure 2, after the polymerization reaction, the reaction mixture is being transferred through the tube 3 from the vessel 1 into the vessel 2 by means of the pressure of the gas entering the vessel 1 through the tube 5. Thus, the valve 7 is activated, allowing gas to flow from the tube 14 into the vessel 1. The valve 8 for its part is deactivated, allowing excess gas to flow out. A suitable gas pressure is preferably 0.1 - 1. atm.

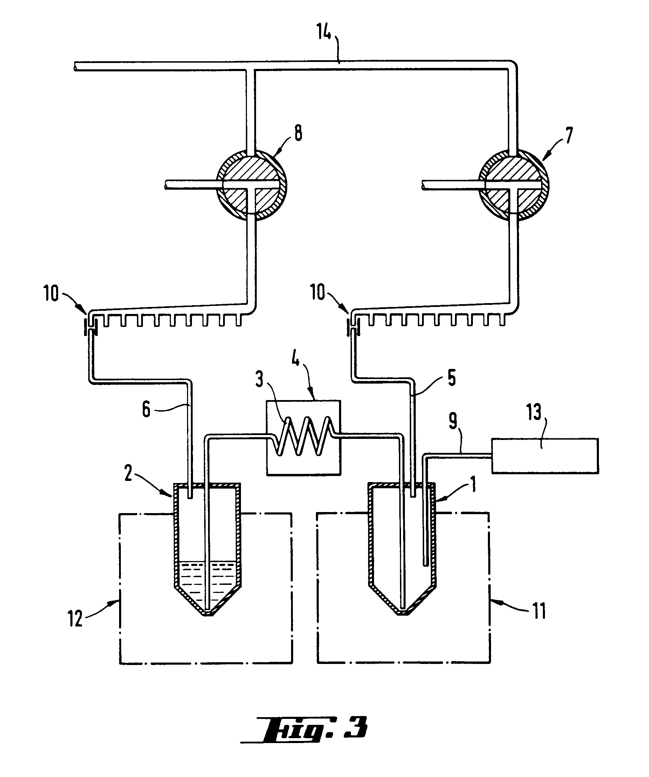

In Figure 3, denaturation is occurring in the vessel 2, the temperature of which has been adjusted to a suitable value by means of a heat exchanger 12. Each valve 7 and 8 is deactivated, and so the transfer of fluids from one vessel into the other is not possible.

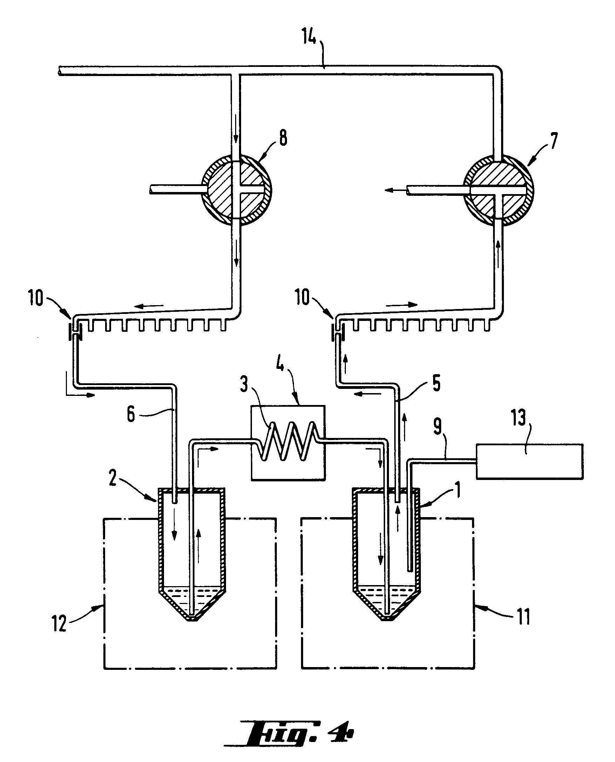

In Figure 4, the reaction mixture is being transferred back from the vessel 2 into the vessel 1. The valve 8 is activated, allowing gas to flow into the vessel 2. Under the pressure of the gas, the fluid tends to pass into the vessel 1. The transfer is preferably carried out at such a speed that there is time for the hybridization reaction to occur in the heat exchanger. The valve 7 is deactivated so that excess gas can flow out to make space for the fluid flowing into the vessel 1. When necessary, polymerase is added through the tube 9.

The steps depicted in Figures 1-4 can be repeated several, even tens of times under microprocessor control, and so the amplification occurs automatically from beginning to end.

The invention is not limited in any way to the preferred embodiment described above and depicted in Figures 1-4; many variations are possible within the inventional idea. In the apparatus described above it is possible, for example, to use a vacuum instead of elevated pressure.

An amplification apparatus in which the transfer of fluid is effected by means of elevated pressure or a vacuum conveniently enables several target nucleic acid mixtures to be treated simultaneously, since one and the same valve can be used for controlling the transfer of the reaction mixtures in all vessel pairs from one vessel to the other. The reaction mixture can also be transferred from one vessel to the other by means of a fluid pump, in which case the gas tubes 5 and 6 are not necessary. In such a case it is possible, for example, to use a pump with a reversible flow direction, in which case only one tube 3 is required between the vessels 1 and 2. The above amplification of nucleic acids can also be performed on complementary DNA obtained from ribonucleic acid by using a reverse transcriptase.

The series of amplification reactions is preferably started with the step according to Figure 1, i.e. the reagents necessary for the amplification and a single-stranded target nucleic acid are incubated at the optimum temperature for the optimum period in order to perform the polymerase reaction. If the target nucleic acid is originally double-stranded, it is rendered single-stranded before the first step. It is, of course, possible to use the amplification apparatus for the denaturation of the target nucleic acid, in which case the whole reaction series is started with the step depicted in Figure 3. In this case it is in general advantageous to use a longer denaturation period than during actual amplification.

The optimum temperatures and times to be used in the reaction series are determined on the basis of the target nucleic acid, as well as the primer and polymerase used. An expert in the field is himself able to adjust the apparatus and to select the suitable conditions for the amplification reactions to be performed at a given time.

A number of different procedures are possible with respect to the addition of the polymerase. The enzyme may be introduced either continuously or intermittently into the vessel in which the polymerase reaction takes place. If the enzyme is thermostable, it is incorporated into the reaction mixture at the beginning of the reaction series and added thereafter only when needed. No enzyme needs to be added if it has been introduced into the reaction vessel either in an immobilized form or in a suitable slowly releasing dosage form. If the enzyme is immobilized, it of course does not pass from the reaction vessel. When a slowly releasing dosage form is used, it is important that the release of the polymerase is regulated so that its concentration remains suitable for the duration of as many reaction cycles as is necessary.

The temperature of the heat exchanger is determined on the basis of the length and nucleotide sequence of the primer used. When the hybridization temperature of the primer differs considerably from the optimum temperature of the polymerization enzyme, the temperature profile of the reaction mixture is preferably regulated so that the primer has time to hybridize as early as in the heat exchanger. The thermolability of the polymerization enzyme can also be taken into consideration in the regulation of the heat exchanger by making sure that the reaction mixture is cooled sufficiently before it comes into contact with the enzyme. The retention time in the heat exchanger of the solution which contains the denatured nucleic acid can be regulated by means of the tube length and the pressure of the gas introduced into the denaturation vessel, or respectively by adjusting the vacuum or the efficiency of the fluid pump.

## Claims

1. An apparatus for the amplification of nucleic acids, **characterized** by one or several pairs of vessels (1, 2) interconnected by a tube or tubes (3), thermoregulators (11, 12) regulating the temperatures of the vessels (1 and 2, respectively), and by a fluid transfer system for transferring fluid from one vessel (1) into the other vessel (2) or vice versa.

2. An apparatus according to Claim 1, **characterized** in that the fluid transfer system includes gas tubes (5, 6) leading to the vessels (1, 2) in the vessel pair or vessel pairs and valves (7, 8) which regulate the gas flow.

3. An apparatus according to Claim 1 or 2, **characterized** in that the tube (3) runs via a separate heat exchanger (4).

4. An apparatus according to any one of Claim 1 to 3, **characterized** in that it additionally includes a dosing device (13) and tube (9) for the adding of reagents and/or sample.

5. An apparatus according to any one of Claims 2 to 4, **characterized** in that the operation of the valves (7, 8) is microprocessor-controlled.

6. An apparatus according to any one of Claims 2 to 5, **characterized** in that the gas tubes (5, 6) are connected to gas distributors (10) which are controlled by valves (7, 8).

7. An apparatus according to any one of Claims 1 to 6, **characterized** in that the tube (3) interconnecting the vessels (1, 2) extends all the way to the bottom of the vessels.

8. An apparatus according to any one of Claims 1 to 7, **characterized** in that the bottom of each of the vessels (1, 2) converges downwards.

9. An apparatus according to any one of Claims 4 to 8, **characterized** in that the reagent-dosing device works continuously.

**10.** An apparatus according to any one of Claims 4 to 8, **characterized** in that the reagent-dosing device (13) works intermittently.

**11.** A method for the amplification of nucleic acids by performing the following steps:

a) a reaction mixture containing the reagents necessary in the polymerization reaction and a single-stranded target nucleic acid is incubated

b) any double-stranded nucleic acid present in the reaction mixture is denatured by heating

c) the reaction mixture is cooled

d) if necessary, DNA polymerase is added

e) the same reaction cycle is repeated the number of times suitable in the given case,

**characterized** in that steps a) and b) are performed in separate vessels of an apparatus according to anyone of claims 1-10 and that the temperturon of the vessels are maintained at all times at levels suitable for the given steps.

**12.** A method according to Claim 11, **characterized** in that the denatured nucleic acid is cooled before being transferred into the vessel in which the polymerization occurs.

**13.** A method according to Claim 11 or 12, **characterized** in that the transfer of the reaction fluid from one vessel into the other is carried out using gas pressure.

**14.** A method according to any one of Claims 11 to 13, **characterized** in that the polymerase is added continuously.

**15.** A method according to any one of Claims 11 to 13, **characterized** = in that the polymerase is added intermittently.

**16.** A method according to any one of Claims 11 to 15, **characterized** in that the polymerase is in immobilized form in the reaction vessel.

**17.** A method according to any one of Claims 11 to 16, **characterized** in that the polymerase is in a slowly releasing dosage form in the reaction vessel.

**18.** A disposable part of the apparatus according to Claims 1 or 2, **characterized** in that the part includes a vessel pair made up of vessels (1, 2) interconnected by a tube (3), as well as gas tubes (5, 6) leading to the vessels (1, 2).

**Revendications**

**1.** Appareil pour l'amplification des acides nucléiques, caractérisé par une ou plusieurs paires de récipients (1,2) reliés entre eux par un tube ou des tubes (3), des régulateurs de température (11, 12) qui régulent les températures des récipients (1, 2, respectivement) et par un système de transfert de fluide pour transférer un fluide d'un récipient (1) dans l'autre récipient (2) ou inversement.

**2.** Appareil selon la revendication 1, caractérisé en ce que le système de transfert de fluide comprend des tubes à gaz (5,6) conduisant aux récipients (1, 2) de la paire de récipients ou des paires de récipients et des vannes (7, 8) qui régulent le courant de gaz.

**3.** Appareil selon la revendication 1 ou 2, caractérisé en ce que le tube (3) traverse un échangeur de chaleur (4) séparé.

**4.** Appareil selon l'une quelconque des revendications 1 à 3, caractérisé en ce qu'il comprend en outre un dispositif de dosage (13) et un tube (9) pour l'addition de réactifs et/ou d'un échantillon.

**5.** Appareil selon l'une quelconque des revendications 2 à 4, caractérisé en ce que le fonctionnement des vannes (7, 8) est commandé par un microprocesseur.

**6.** Appareil selon l'une quelconque des revendications 2 à 5, caractérisé en ce que les tubes à gaz (5, 6) sont reliés à des distributeurs de gaz (10) qui sont commandés par les vannes (7,8).

**7.** Appareil selon l'une quelconque des revendications 1 à 6, caractérisé en ce que le tube (3) qui relie entre eux les récipients (1, 2) s'étend toujours jusqu'au fond des récipients.

**8.** Appareil selon l'une quelconque des revendications 1 à 7, caractérisé en ce que le fond de chacun des récipients (1,2) converge vers le bas.

**9.** Appareil selon l'une quelconque des revendications 4 à 8, caractérisé ence que le dispositif de dosage de réactifs fonctionne continuellement.

**10.** Appareil selon l'une quelconque des revendications 4 à 8, caractérisé en ce que le dispositif de dosage de réactifs (13) fonctionne par intermittence.

**11.** Procédé pour l'amplification des acides nucléiques par l'accomplissement des étapes suivantes :

a) un mélange réactionnel contenant les réactifs nécessaires pour la réaction de polymérisation et un acide nucléique cible simple brin est incubé

b) tout acide nucléique double brin présent dans le mélange réactionnel est dénaturé par chauffage

c) le mélange réactionnel est refroidi

d) si nécessaire, de l'ADN polymérase est ajoutée

e) le même cycle de réaction est répété un nombre de fois convenable dans le cas donné,

caractérisé en ce que les étapes a) et b) sont accomplies dans des récipients séparés d'un appareil selon l'une quelconque des revendications 1 à 10 et en ce que les températures des récipients sont maintenues à chaque instant à des niveaux appropriés pour les étapes données.

**12.** Procédé selon la revendication 11, caractérisé en ce que l'acide nucléique dénaturé est refroidi avant d'être transféré dans le récipient dans lequel la polymérisation a lieu.

**13.** Procédé selon la revendication 11 ou 12, caractérisé en ce que le transfert du fluide réactionnel d'un récipient dans l'autre est effectué à l'aide d'une pression de gaz.

**14.** Procédé selon l'une quelconque des revendications 11 à 13, caractérisé en ce que la polymérase est ajoutée continuellement.

**15.** Procédé selon l'une quelconque des revendications 11 à 13, caractérisé en ce que la polymérase est ajoutée par intermittence.

**16.** Procédé selon l'une quelconque des revendications 11 à 15, caractérisé en ce que la polymérase est sous forme immobilisée dans le récipient de réaction.

**17.** Procédé selon l'une quelconque des revendications 11 à 16, caractérisé en ce que la polymérase est sous une forme de dosage à libération lente dans le récipient de réaction.

**18.** Partie jetable de l'appareil selon les revendications 1 ou 2, caractérisée en ce que la partie comprend une paire de récipients constituée par des récipients (1, 2) reliés entre eux par un tube (3) et des tubes à gaz (5, 6) conduisant aux récipients (1,2).

**Patentansprüche**

**1.** Vorrichtung zur Amplifikation von Nukleinsäuren, **dadurch gekennzeichnet,** daß ein Paar oder mehrere Paare von Behältern (1, 2) über ein Rohr oder mehrere Rohre (3), Thermoregler (11, 12), die die Temperaturen der Behälter (1 bzw. 2) regeln, und durch ein Fluidtransportsystem zum Transportieren von Fluid von einem Behälter (1) in den anderen Behälter (2) oder umgekehrt, miteinander verbunden sind.

**2.** Vorrichtung nach Anspruch 1, **dadurch gekennzeichnet,** daß das Fluidtransportsystem Gasrohre (5, 6), die zu den Behältern (1, 2) in dem Behälterpaar oder den Behälterpaaren führen, und Ventile (7, 8) aufweist, die den Gasstrom regeln.

**3.** Vorrichtung nach Anspruch 1 oder 2, **dadurch gekennzeichnet,** daß das Rohr (3) über einen getrennten Wärmetauscher (4) geführt ist.

**4.** Vorrichtung nach einem der Ansprüche 1 bis 3, **dadurch gekennzeichnet,** daß zusätzlich eine Dosiervorrichtung (13) und ein Rohr (9) zur Zugabe von Reagenzien und/oder einer Probe vorgesehen sind.

**5.** Vorrichtung nach einem der Ansprüche 2 bis 4, **dadurch gekennzeichnet,** daß der Betrieb der Ventile (7, 8) mikroprozessorgesteuert ist.

**6.** Vorrichtung nach einem der Ansprüche 2 bis 5, **dadurch gekennzeichnet,** daß die Gasrohre (5, 6) mit Gasverteilern (10) verbunden sind, die durch Ventile (7, 8) gesteuert werden.

**7.** Vorrichtung nach einem der Ansprüche 1 bis 6, **dadurch gekennzeichnet,** daß das die Behälter (1, 2) verbindende Rohr (3) sich ganz bis zum Boden der Behälter erstreckt.

**8.** Vorrichtung nach einem der Ansprüche 1 bis 7, **dadurch gekennzeichnet,** daß der Boden jedes Behälters (1, 2) sich nach unten verengt.

**9.** Vorrichtung nach einem der Ansprüche 4 bis 8, **dadurch gekennzeichnet,** daß die Reagenz-Dosiervorrichtung kontinuierlich arbeitet.

**10.** Vorrichtung nach einem der Ansprüche 4 bis 8, **dadurch gekennzeichnet,** daß die Reagenz-Dosiervorrichtung (13) intermittierend arbeitet.

**11.** Verfahren zur Amplifikation von Nukleinsäuren mit den folgenden Verfahrensschritten:

a) Inkubieren eines Reaktionsgemisches enthaltend die zur Polymerisation erforderlichen Reagenzien und eine einsträngige Ziel-Nukleinsäure,

b) Denaturieren einer doppelsträngigen Nukleinsäure, die in dem Reaktionsgemisch enthalten ist, durch Erwärmen,

c) Kühlen des Reaktionsgemisches,

d) gegebenenfalls Zugabe von DNA-Polymerase,

e) Wiederholen des gleichen Reaktionszyklus so oft, wie im gegebenen Fall zweckmäßig ist,

**dadurch gekennzeichnet,** daß die Verfahrensschritte a) und b) in getrennten Behältern einer Vorrichtung nach einem der Ansprüche 1 bis 10 ausgeführt werden,und daß die Temperatur der Behälter über die gesamte Zeit auf Werten gehalten wird, die für die gegebenen Verfahrensschritte geeignet sind.

12. Verfahren nach Anspruch 11, **dadurch gekennzeichnet,** daß die denaturierte Nukleinsäure vor dem Transport in den Behälter, in dem die Polymerisation erfolgt, gekühlt wird.

13. Verfahren nach Anspruch 11 oder 12, **dadurch gekennzeichnet,** daß der Transport des Reaktionsfluids aus einem Behälter in den anderen unter Gasdruck erfolgt.

14. Verfahren nach einem der Ansprüche 11 bis 13, **dadurch gekennzeichnet,** daß die Polymerase kontinuierlich zugeführt wird.

15. Verfahren nach einem der Ansprüche 11 bis 13, **dadurch gekennzeichnet,** daß die Polymerase intermittierend zugeführt wird.

16. Verfahren nach einem der Ansprüche 11 bis 15, **dadurch gekennzeichnet,** daß die Polymerase in dem Reaktionsbehälter in immobilisierter Form vorliegt.

17. Verfahren nach einem der Ansprüche 11 bis 16, **dadurch gekennzeichnet,** daß die Polymerase im Reaktionsbehälter in einer langsam frei werdenden Dosierung vorliegt.

18. Einwegteil der Vorrichtung nach Anspruch 1 oder 2, **dadurch gekennzeichnet,** daß das Teil ein Behälterpaar aus durch ein Rohr (3) verbundenen Behältern (1, 2) sowie Gasrohre (5, 6), die zu den Behältern (1, 2) führen, umfaßt.

**Fig. 1**

Fig. 2

Fig. 3

Fig. 4